# EUROPEAN PATENT SPECIFICATION

(11) **EP 1 734 936 B1**
(45) Date of publication and mention of the grant of the patent: **10.09.2014**
(21) Application number: 05712364.8
(22) Date of filing: 31.01.2005
(51) Int. Cl.: A61K 9/127, A61K 31/74, A61K 31/785, A61K 31/795, A61K 47/48

(54) **AMPHIPHILIC STAR-LIKE OR SCOPRION-LIKE MACROMOLECULES, VARIOUS COMPOSITIONS AND USES THEREOF**
AMPHIPHILE STERNFÖRMIGE ODER SKORPIONFÖRMIGE MAKROMOLEKÜLE, VERSCHIEDENE ZUSAMMENSETZUNGEN UND IHRE VERWENDUNGEN
MACROMOLECULES AMPHIPHILES EN FORME D'ÉTOILE OU DE SCORPION, LES COMPOSITIONS VARIÉES ET LEUR EMPLOI

(30) Priority: 30.01.2004 US 540867 P; 30.01.2004 US 540765 P
(43) Date of publication of application: 27.12.2006
(73) Proprietor: Rutgers, The State University, New Brunswick, NJ 08903 (US)
(72) Inventor: UHRICH, Kathryn, E., Plainfield, NJ 07060 (US); MOGHE, Prabhas, Basking Ridge, NJ 07920 (US)
(74) Representative: Grünecker, Kinkeldey, Stockmair & Schwanhäusser
(86) International application number: PCT/US2005/002900
(87) International publication number: WO 2005/074887

(56) References cited:
- DJORDJEVIC-J. MICHNIAK-B. UHRICH-K-E.: "Amphiphilic Star-Like Macromolecules as Novel Carriers for Topical Delivery of Nonsteroidal Anti-Inflammatory Drugs" AAPS PHARMSCI, vol. 5, no. 4, 2003, pages 1-12, XP002335359
- HONGBO LIU, ANNA JIAN, JIAN GUO, KATHRYN E. UHRICH: "Unimolecular Micelles: Synthesis and Characterization of Amphiphilic Polymer Systems" JOURNAL OF POLYMER SCIENCE: PART A: POLYMER CHEMISTRY, vol. 37, 1999, pages 703-711, XP009050403
- CHNARI E ET AL: "Nanoscale anionic macromolecules for selective retention of low-density lipoproteins" BIOMATERIALS, ELSEVIER SCIENCE PUBLISHERS BV., BARKING, GB, vol. 26, no. 17, June 2005 (2005-06), pages 3749-3758, XP004696124 ISSN: 0142-9612
- LU TIAN: "Novel Amphiphilic Macromolecules for Drug Delivery Applications: Design, Synthesis and Characterization." 2004, DISSERTATION , NEW BRUNSWICK, NEW JERSEY , XP001206960 page 13 - page 48 page 114 - page 138 page 160 - page 175

## Description

### Background of the Invention

A method for the synthesis of amphiphilic star-like macromolecules (ASMs) and amphiphilic scorpion-like macromolecules (AScMs) and their use as agents for drug delivery has been described.

Low density lipoprotein (LDL) is a major carrier of cholesterol in blood. LDL combines with cholesterol to form LDL-cholesterol, a large spherical particle, the core of which contains about 1,500 molecules of esterified cholesterol. Cholesterol is essential for the growth and viability of cells in higher organisms. High serum levels of cholesterol, however, are believed to be associated with disease and death by contributing to the formation of atherosclerotic plaques in arteries throughout the body. Accordingly, elevated serum levels of LDL-cholesterol have been employed as predictors and risk factors of cardiovascular disease and atherosclerosis. The oxidative modification of LDL also has been reported to play a significant role in mediating the atherogenic process. Thus, the sequestration and/or removal of LDL is not only important for lowering plasma cholesterol, but also to inhibit its oxidative modification and, therefore, for preventing atherosclerosis and its complications.

Accordingly, there is a need for products, compositions, *e.g*. compositions comprising nanoscale particles, and methods suitable for *in vivo*, *ex vivo* and *in vitro* sequestering and/or removing agents such as Low Density Lipoproteins (LDL), *e.g*., unoxidized and weakly oxidized LDL.

Djordjevic J., Michniak B., Uhrich K. E.; Amphiphilic Star-Like Macromolecules as Novel Carriers for Topical Delivery of Nonsteroidal Anti-Inflammatory Drugs, AAPS PharmSci. 2003, 5, 4, 1-12 evaluates amphiphilic star-like macromolecules (ASMs) as a topical drug delivery system, in which indomethacin, piroxicam, and ketoprofen are individually encapsulated into the ASMs using co-precipitation.

Liu H., Jiang A., Guo J., Uhrich K- E., Unimolecular micelles: Synthesis and characterization of amphiphilic polymer systems, Journal of Polymer Science Part A: Polymer Chemistry, 1999, 37, 6, 703-711, 15 describes the synthesis of unimolecular micelles from mucic acid, fatty acids. and poly(ethylene glycols) to create biocompatible polymers.

Chnari E., Lari H. B., Tian L., Uhrich K. E., Moghe P. V.; Nanoscale anionic macromolecules for selective retention of low-density lipoproteins, Biomaterials, 2005, 26(17), 3749-58 discloses synthetically designed anionic nanocarriers that mimic the charge properties of glycosaminoglycans can potentially sequester low-density lipoproteins (LDL) during the treatment of atherosclerosis.

Tian L., Novel Amphiphilic Macromolecules for Drug Delivery Applications: Design, Synthesis and Characterization, May 2004, 1-180 discloses the design, synthesis, and characterization of biocompatible amphiphilic macromolecules for biomedical applications such as drug delivery and tissue engineering.

### Summary of the Invention

The inventors have discovered that amphiphilic macromolecules (e.g., AScMs and ASMs) sequester low-density lipoproteins (LDL), *e.g*. unoxidized and/or weakly oxidized LDL, and are useful, for example, in the inhibition of atherosclerotic development.

The invention provides a method for sequestering and/or removing LDL comprising contacting a medium comprising LDL with a sequestering and/or removing effective amount of a compound of chemical formula (I)

A-X-Y-Z-R₁ (I)

wherein A comprises a carboxy group or is absent;
X comprises a polyol, wherein one or more polyol hydroxyls are substituted by acyl;
Y comprises -C(=O)-,-C(=S)-, or is absent;
Z comprises O, S or NH; and
R₁ comprises a polyether.

The invention also provides a method for inhibiting atherosclerosis or atherosclerotic development, which is conducted by contacting or administering ananti-atherosclerosis or anti-atherosclerotic development amount of a compound of formula (I).

The invention further provides the use of a compound of chemical formula (I) to prepare a medicament useful for sequestering and/or removing agents, such as LDL, in and/or from an animal, and for inhibiting atherosclerotic development in an animal.

The invention also provides a method for sequestering and/or removing LDL comprising contacting a medium comprising LDL with a sequestering and/or removing effective amount of a compound of

R (-O-R₁), and R(-NH-R₁)ₓ

wherein R(-O-)ₓ is a polyol moiety and R (-NH)ₓ is a polyamine moiety, with x being between 2 and 10, inclusive, and each R₁ independently has the structure: 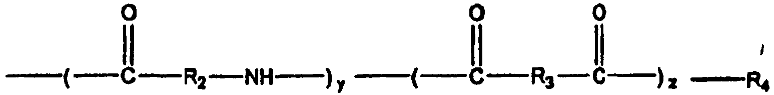 wherein 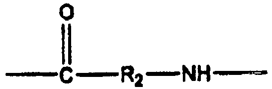 is a divalent amino acid moiety with R₂ being a covalent bond or having from 1 to 8 carbon atoms, and y and z are between 0 and 10, inclusive, provided that y and z are not both 0;
wherein 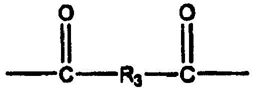 is a divalent dicarboxylic acid moiety in which R₃ is an alkylene or cycloalkylene group containing from 1 to 15 carbon atoms, substituted with a total of from 1 to 10 hydroxyl groups, with at least a portion of the hydroxyl groups being acylated with from 3 to 24 carbon atom carboxylic acids; and wherein R₄ is a poly (alkylene oxide) having the structure:

R₅ - (R₆-O-) a - R₆-Q-

with R₅ being selected from the group consisting of 1 to 40 carbon atom alkyl groups, -OH, -OR₇, -NH₂, -NHR₇, -NHR₇R₈, -C-OH, -C-OR₇, -C-O-C-R₇, -C-NH₂, -C-NHR₇ and -C-NR₇R₈; R₆, R₇ AND R₈ being independently selected from the group consisting of 2 to 40 carbon atom, straight-chain or branched alkylene groups;
Q being a divalent linkage moiety; and
a being between 2 and 110, inclusive.

The invention also provides the use of compound of formula R (-O-R₁) or R(-NH-R₁)ₓ to prepare a medicament useful for sequestering and/or removing LDL.

The invention also provides a method for sequestering and/or removing LDL comprising contacting a medium comprising LDL with a sequestering and/or removing effective amount of a compound of formula (II):

R¹-((R²)ₐ-(R)_{b}-(R⁴)_{c}-(R⁵)_{d}-(R⁶)ₑ)ₙ (II)

wherein R¹ is a core comprising a polyol or polyacid;
each R² independently is a divalent or polyvalent group having the formula - X¹-R⁸-(X^{1a})_{g}-, wherein X¹ and X^{1a} are independently -C(=O)-, -C(=S)-, -O-, -S-, - N(R⁷)- or absent, and each R^{g} is independently -(C₁₋₈)alkylene-, branched -(C₁₋₈)alkylene- or -(C₆₋₁₀)aryl-; a is 0 or an integer from 1 to 10; and g is an integer from 1 to 6;
each R³ independently is a divalent dicarboxylic acid moiety having the formula -C(=O)-R⁹-C(=O)-, wherein R⁹ is an alkylene or cycloalkylene group containing from 1 to 15 carbon atoms, substituted with a total of from 1 to 10 hydroxy groups, wherein one or more of the hydroxy groups of the dicarboxylic acid are acylated with an acid residue; and b is an integer from 1 to 10;
each R⁴ independently is a divalent or polyvalent group having the formula -X²-R¹⁰-(X^{2a})ₕ-, wherein X² is -C(=O)-, -C(=S)-, -O-, -S-, -N(R⁷)- or absent; X^{2a} is -C(=O)-, -C(=S)-, -O-, -S-, or -N(R⁷)- and R¹⁰ is -(C₁₋₈)alkylene-, branched -(C₁₋₈)alkylene- or -(C₆₋₁₀)aryl-; and c is 0 or an integer from 1 to 10; and h is an integer from 1 to 6;
each R⁵ independently is a group having the formula:

   -R¹²-(R¹¹)_{f}-R¹²-X³-

   wherein R¹¹ is a sugar moiety; or a poly(alkylene oxide) or poly(alkylene imine) group having the formula -(-X⁴-R¹³)-; wherein R¹³ is -(C₂₋₄₀)alkylene- or branched -(C₃₋₄₀)alkylene-; wherein each X³ is independently -C(=O)-, -C(=S)-, -O-, -S-, -N(R⁷)- or absent; each X⁴ is independently -O-, or -N(R⁷)-; and f is an integer from 2 to 150; and d is from 1 to 6;
   each R¹² is independently a bond, -(C₁₋₄₀)alkylene- or branched -(C₁₋₄₀)alkyle-groups, wherein each R¹² is optionally substituted with one or more (e.g., 1, 2, or 3) functional group; and X⁴ is -O-, -S-, or -N(R⁷)-.

The invention also provides the use of compound of formula II to prepare a medicament useful for sequestering and/or removing LDL.

The invention also provides a method for treating a disease associated with pathological cells in the body of an animal, comprising administering to the animal a therapeutic agent that is associated with an amphiphilic macromolecule that targets the agent to the cells.

The invention also provides the use of a therapeutic agent that is associated with an amphiphilic macromolecule that targets the agent to selected the cell to prepare a medicament useful for treating a disease associated with pathological cells in the body of an animal.

The invention also provides a conjugate comprising, one or more targeting agents linked to a chemical of formula (I):

A-X-Y-Z-R₁ (I)

wherein A comprises a carboxy group or is absent; X comprises a polyol, wherein one or more polyol hydroxyls are substituted by acyl; Y comprises -C(=O)-,-C(=S)-, or is absent; Z comprises O, S or NH; and R₁ comprises a polyether.

The invention also provides a method for sequestering LDL *in vitro* or *in vivo* comprising contacting LDL with an effective sequestering amount of a compound of formula (I):

A-X-Y-Z-R₁ (I)

wherein: A is a carboxy group or is absent; X is a polyol; Y is -C(=O)-,-C(=S)-, or is absent; Z is O, S or NH; and R₁ is a polyether, wherein one or more hydroxy groups of the polyol are acylated with a fatty acid residue. In one embodiment, the LDL is sequestered from an animal by administering the effective sequestering amount of the compound of formula (I) to the animal.

The invention also provides a method for inhibiting atherosclerotic development in an animal comprising administering to the animial an effective atherosclerotic development inhibiting amount of a compound of formula (I):

A-X-Y-Z-R₁ (I)

wherein: A is a carboxy group or is absent; X is a polyol; Yis -C(=O)-,-C(=S)-, or is absent; Z is O, S or NH; and R₁ is a polyether, wherein one or more hydroxy groups of the polyol are acylated with a fatty acid residue.
The invention is further directed to the following embodiments 1 to 87.
1. A method for sequestering and/or removing LDL comprising contacting a medium comprising LDL with a sequestering and/or removing effective amount of a compound of chemical formula (I)

   A-X-Y-Z-R₁ (I)

   wherein A comprises a carboxy group or is absent;
   X comprises a polyol, wherein one or more polyol hydroxyls are substituted by acyl;
   Y comprises -C(=O)-,-C(=S)-, or is absent;
   Z comprises O, S or NH; and
   R₁ comprises a polyether.
2. The method of embodiment 1, wherein in the polyol acyl comprises a fatty acid(s).
3. The method of embodiment 1, wherein the LDL is sequestered and/or removed by in vitro, ex vivo, or in vivo administration of the compound of formula (I).
4. A method for inhibiting atherosclerosis or atherosclerotic development, comprising the method of embodiment 1, which is conducted by contacting or administering ananti-atherosclerosis or anti-atherosclerotic development amount of a compound of formula (I).
5. The method of embodiment 2 or 3, wherein the compound is contacted with, or administered to an animal.
6. The method of embodiment 5, wherein the animal comprises a mammal.
7. The method of embodiment 6, wherein the mammal comprises a human.
8. The method of any one of embodiments 1-7, wherein the polyol comprises a (C₂-C₂₀) alkyl polyol.
9. The method of any one of embodiments 1-8, wherein the polyol comprises 2 to 20 hydroxyl groups.
10. The method of any one of embodiments 1-9, wherein the polyol is substituted with one or more acyl.
11. The method of any one of embodiments 1-10, wherein the polyol comprises a mono-or dicarboxylic (C₂-C₂₀) alkyl polyol substituted with 1 to 10 hydroxyl(s).
12. The method of any one of embodiments 1-11, wherein the polyol comprises one or more of mucic acid, malic acid, citromalic acid, alkylmalic acid, hydroxy glutaric acid derivatives, alkyl glutaric acids, tartaric acid, or citric acid.
13. The method of any one of embodiments 1-12, wherein the polyol comprises one or more of 2,2-(bis(hydroxymethyl)propionic acid, tricine, or a saccharide.
14. The method of any one of embodiments 1-13, wherein the polyether comprises 2 to 150 alkylene oxide units.
15. The method of embodiments 1-14, wherein each alkylene oxide unit comprises straight or branched (C₂-C₄) alkylene oxide.
16. The method of embodiments 1-15, wherein the polyether comprises an alkoxy-terminal group.
17. The method of any one of embodiments 1-16, wherein the polyether is linked to the polyol through a linker comprising ester, thioester, or amide.
18. The method of any one of embodiments 1-17, wherein the polyether comprises the chemical formula

   R₅-(R₆-O-)ₐ-R₆-Q- (II),

   wherein
   R₅ comprises straight or branched (C₁-C₂₀) alkyl, -OH, -OR₇, -NH₂, -NHR₇, -NHR₇R₈, - CO₂H, -SO₃H (sulfo), -CH₂-OH, -CH₂-OR₇, -CH₂-O-CH₂-R₇, -CH₂-NH₂, -CH₂-NHR₇, -CH₂-NR₇R₈, -CH₂CO₂H, -CH₂SO₃H, or -O-C(=O)-CH₂-CH₂-C(=O)-O-;
   R₆ comprises straight or branched divalent (C₂-C₁₀) alkylene;
   each R₇ and R₈ comprises, independently, straight or branched (C₁-C₆) alkylene;
   Q comprises -O-, -S-, or -NR₇; and
   a comprises an integer of 2 to 110, inclusive.
19. The method of any one of embodiments 1-18, wherein the polyether comprises a polyethylene glycol comprising a methoxy terminal group.
20. The method of embodiment 2-19, wherein the fatty acid(s) comprise(s) (C₂-C₂₄) fatty acid(s).
21. The method of any one of embodiments 2-20, wherein the fatty acid(s) comprise(s) one or more of caprylic, capric, lauric, myristic, myristoleic, palmitic, palmitoleic, stearic, oleic, linoleic, arachidic, behenic, or erucic acid.
22. The method of any one of embodiments 1-21 wherein the compound of formula (I) has the chemical structure 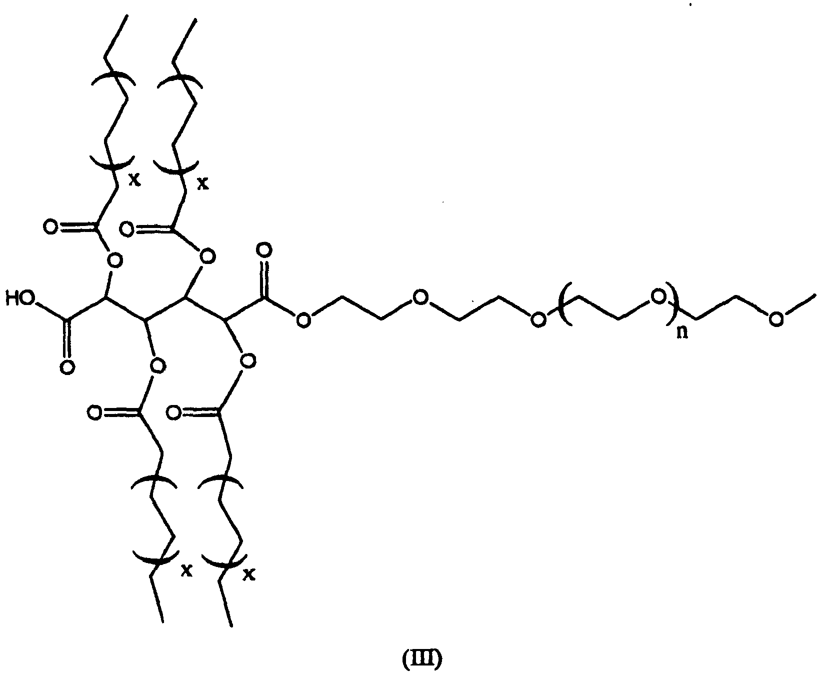 wherein each x comprises, independently, 1, 2, 3, or 4; and n is 36.
23. The method of anyone of embodiments 1-22, wherein the compound of chemical formula (I) or (II) is provided in the form of a nanoparticulate formulation.
24. The use of any one of the compounds of formula (I), (II), or (III) as described in any one of embodiments 1-22 to prepare a medicament useful for inhibiting or reducing atherosclerosis or atherosclerotic development in an animal.
25. The use of embodiment 23 or 24, wherein the animal comprises a mammal.
26. The use of embodiment 23 or 24, wherein the mammal comprises a human.
27. A method for sequestering and/or removing LDL comprising contacting a medium comprising LDL with a sequestering and/or removing effective amount of a compound of

   R (-D-R₁), and R(-NH-R₁)ₓ

   wherein R(-O-)ₓ is a polyol moiety and R (-NH)ₓ is a polyamine moiety, with x being between 2 and 10, inclusive, and each R₁ independently has the structure: 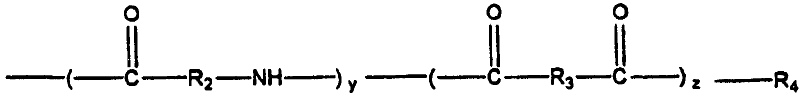 wherein 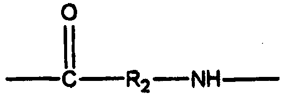 is a divalent amino acid moiety with R₂ being a covalent bond or having from 1 to 8 carbon atoms, and y and z are between 0 and 10, inclusive, provided that y and z are not both 0;
   wherein 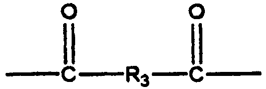 is a divalent dicarboxylic acid moiety in which R₃ is an alkylene or cycloalkylene group containing from 1 to 15 carbon atoms, substituted with a total of from 1 to 10 hydroxyl groups, with at least a portion of the hydroxyl groups being acylated with from 3 to 24 carbon atom carboxylic acids; and wherein R₄ is a poly (alkylene oxide) having the structure:

   Rs - (R₆-O-) a - R₆-Q-

   with R₅ being selected from the group consisting of 1 to 40 carbon atom alkyl groups, -OH, -OR₇, -NH₂, -NHR₇, -NHR₇R₈, -C-OH, -C-OR₇, -C-O-C-R₇. -C-NH₂, -C-NHR₇ and -C-NR₇R₈; R₆, R₇ AND R₈ being independently selected from the group consisting of 2 to 40 carbon atom, straight-chain or branched alkylene groups;
   Q being a divalent linkage moiety; and
   a being between 2 and 110, inclusive.
28. The method of embodiment 27, wherein in the polyol acyl comprises a fatty acid(s).
29. The method of embodiment 27, wherein the LDL is sequestered and/or removed by *in vitro*, *ex vivo*, or *in vivo* administration of the compound.
30. A method for inhibiting atherosclerosis or atherosclerotic development, comprising the method of embodiment 27, which is conducted by contacting or administering an anti-atherosclerosis or anti-atherosclerotic development amount of the compound.
31. The method of embodiment 27, wherein x is a 3 or 4.
32. The method of embodiment 31, wherein the compound has the structure R(-NH-R₁)ₓ, wherein R(-NH-) is a polyamine moiety.
33. The method of embodiment 31, wherein the compound has the structure R(-O-R₁)ₓ, wherein R(-O-)ₓ is a polyoyl moiety.
34. The method of embodiment 33, wherein said polyoyl moiety is an aromatic polyoyl moiety.
35. The method of embodiment 34, wherein said polyoyl moiety is a 1, 1, 1-tris (hydroxyphenyl) ethane moiety.
36. The method of embodiment 27, wherein every hydroxyl group said divalent dicarboxylic acid moiety is acylated with a 6 to 24 carbon atom carboxylic acid group.
37. The method of embodiment 36, wherein y is 0.
38. The method of embodiment 33, wherein said divalent dicarboxylic acid moiety is a mucic acid moiety.
39. The method of embodiment 33 wherein said polyoyl moiety is an aliphatic or cycloaliphatic polyoyl moiety.
40. The method of embodiment 33, wherein said polyoyl moiety is a cyclic crown ether or cyclodextrin moiety.
41. The method of embodiment 27, wherein poly (alkylene oxide) is a methoxy-terminated poly (ethylene glycol) and Q is -NH-.
42. The method of embodiment 27, wherein Q is -O-, or forms an anhydride linkage.
43. A method for sequestering and/or removing LDL comprising contacting a medium comprising LDL with a sequestering and/or removing effective amount of a compound of formula (II):

   R¹-((R²)ₐ-(R³)_{b}-(R⁴)_{c}-(R⁵)_{d}-(R⁶)_{c})ₙ (II)

   wherein R¹ is a core comprising a polyol or polyacid;
   each R² independently is a divalent or polyvalent group having the formula -X¹-R⁸-(X^{1a})₈-, wherein X¹ and X^{1a} are independently -C(=O)-, -C(=S)-, -O-, -S-, -N(R⁷)- or absent, and each R⁸ is independently -(C₁₋₈)alkylene-, branched -(C₁₋₈)alkylene- or -(C₆₋₁₀)aryl-; a is 0 or an integer from 1 to 10; and g is an integer from 1 to 6;
   each R³ independently is a divalent dicarboxylic acid moiety having the formula -C(=O)-R⁹-C(=O)-, wherein R⁹ is an alkylene or cycloalkylene group containing from 1 to 15 carbon atoms, substituted with a total of from 1 to 10 hydroxy groups, wherein one or more of the hydroxy groups of the dicarboxylic acid are acylated with an acid residue; and b is an integer from 1 to 10;
   each R⁴ independently is a divalent or polyvalent group having the formula -X²-R¹⁰-(X^{2a})ₕ-, wherein X² is -C(=O)-, -C(=S)-, -O-, -S-, -N(R⁷)- or absent; X^{2a} is -C(=O)-, -C(=S)-, -O-, -S-, or -N(R⁷)- and R¹⁰ is -(C₁₋₈)alkylene-, branched -(C₁₋₈)alkylene- or -(C₆₋₁₀)aryl-; and c is 0 or an integer from 1 to 10; and h is an integer from 1 to 6;
   each R⁵ independently is a group having the formula:

      -R¹²-(R¹¹)_{f}-R¹²-X³-

      wherein R¹¹ is a sugar moiety; or a poly(alkylene oxide) or poly(alkylene imine) group having the formula -(-X⁴-R¹³)-; wherein R¹³ is -(C₂₋₄₀)alkylene- or branched -(C₃₋₄₀)alkylene-; wherein each X³ is independently -C(=O)-, -C(=S)-, -O-, -S-, -N(R⁷)- or absent; each X⁴ is independently -O-, or -N(R⁷)-; and f is an integer from 2 to 150; and d is from 1 to 6;
      each R¹² is independently a bond, -(C₁₋₄₀)alkylene- or branched -(C₁₋₄₀)alkylene- groups, wherein each R¹² is optionally substituted with one or more (e.g., 1, 2, or 3) functional group; and X⁴ is -O-, -S-, or -N(R⁷)-.
44. The method of embodiment 43, wherein in the polyol acyl comprises a fatty acid(s).
45. The method of embodiment 43, wherein the LDL is sequestered and/or removed by *in vitro*, *ex vivo*, or *in* vivo administration of the compound.
46. A method for inhibiting atherosclerosis or atherosclerotic development, comprising the method of embodiment 43, which is conducted by contacting or administering an anti-atherosclerosis or anti-atherosclerotic development amount of the compound.
47. The method of any one of embodiments 43-46, wherein R² has the formula: 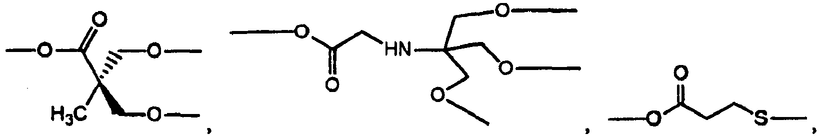 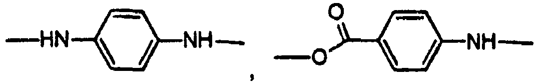 or 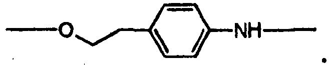
48. The method of any one of embodiments 43-47, wherein R² has the formula: 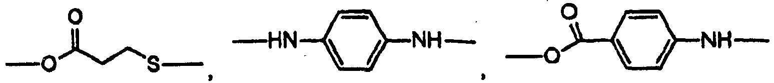 or 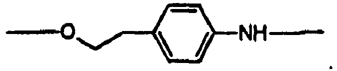
49. The method of any one of embodiments 43-48, wherein R² has the formula: 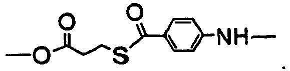
50. The method of any one of embodiments 43-49, wherein the R¹-R² combination has the formula: 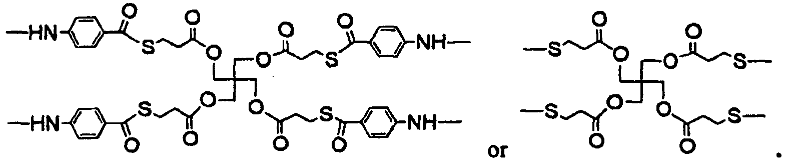
51. The method of any one of embodiments 43-50, wherein R³ has the formula 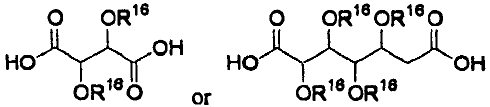 wherein each R¹⁶ is an alkanoyl group having from 2 to 24 carbon atoms.
52. The method of any one of embodiments 43-51, wherein R³ is 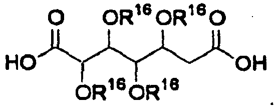
53. The method of any one of embodiments 43-52, wherein R¹⁶ is an alkanoyl group having from 6 to 18 carbon atoms.
54. The method of any one of embodiments 43-53, wherein R¹ has from 2 carbons to 20 carbons.
55. The method of any one of embodiments 43-54, wherein R¹ has from 3 carbons to 12 carbons.
56. The method of any one of embodiments 43-55, wherein the R¹ moeity has from 4 carbons to 10 carbons.
57. The method of any one of embodiments 43-45 or 47-56, wherein R¹ is a cycloaliphatic polyol.
58. The method of any one of embodiments 43-57, wherein R¹ is a polyacid having the formula 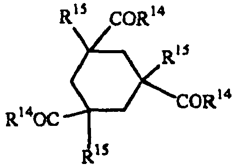 or 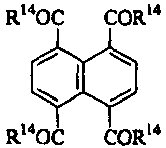 or a polyol having the formula 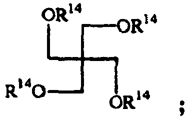 wherein each R¹⁴ is -(R²)ₐ-(R³)_{b}-(R⁴)_{c}-(R⁵)_{d}-(R⁶)_{c}; and wherein R¹⁵ is hydrogen or (C₁₋₆)alkyl; and R², R³, R⁴, R⁵, a, b, c, and d, are as defined hereinabove.
59. The method of embodiment 58, where R¹⁵ is alkyl.
60. The method of embodiment 59, where R¹⁵ is methyl, ethyl , or propyl.
61. The method of embodiment 19, where R¹⁵ is methyl, or propyl.
62. The method of any one of embodiments 43-58, wherein R¹ comprises a core having the formula: 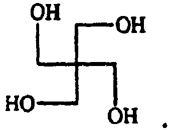
63. The method of any one of embodiments 43-62, wherein R² is -C(=O)-CH₂-CH₂-S-.
64. The method of any one of embodiments 43-58 or 62-63, wherein the R¹-R² combination is pentaerythritol tetrakis(3-mercaptopropionate).
65. The method of any of embodiments 43-58 or 62-63, wherein the R¹ moeity comprises from 2 to 20 hydroxy groups.
66. The method of any one of embodiments 43-58 or 62-65, wherein the R¹ moeity comprises from 2 to 12 hydroxy groups.
67. The method of any one of embodiments 43-58 or 62-66, wherein the R¹ moeity comprises from 2 to 10 hydroxy groups.
68. The method of any one of embodiments 43-67, wherein the R¹ moeity is substituted with one or more carboxy groups.
69. The method of any of embodiments 43-68, wherein the R¹ moeity is substituted with two carboxy groups.
70. The method of any one of embodiments 43-69,wherein the R¹ moeity is substituted with one carboxy group.
71. The method of any one of embodiments 43-70, wherein R⁴ has the formula: 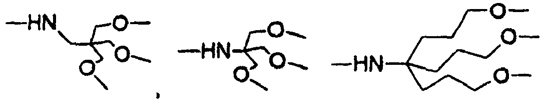
72. The method of any one of embodiments 43-71, wherein R⁵ has the formula:

   -R¹²-(O-R¹³)_{f}-R¹²-,

   wherein R¹³ is a 1 to 20 carbon straight-chain or branched alkyl group,
   wherein each R¹² is optionally substituted with one or more functional groups selected from the group consisting of -OH, -OR^{a}, -NR^{a}R^{b}, -CO₂H, -SO₃H,-CH₂-OR^{a}, -CH₂-CH₂-R^{a}, - CH₂CO₂H, -CH₂SO₃H; -O-C(=O)-CH₂-CH₂-C(=O)-O- or -CH₂-NR^{a}R^{b};
   Q is -O-, -S-, and -NR^{a}- ; and
   R¹² is a 1 to 10 carbon straight-chain or branched divalent alkylene group;
   R^{a} and R^{b} are each independently hydrogen (C₁₋₆)alkyl, aryl, aryl(C₁₋₈)alkylene
   f is an integer from 2 to 150, inclusive.
73. The method of any one of embodiments 43-72, wherein the R⁵ is a polyethylene ether having between 2 and 110 alkylene oxide repeating units.
74. The method of any one of embodiments 43-73, wherein the alkylene oxide units containing from 2 to 10 carbon atoms and may be straight chained or branched.
75. The method of any one of embodiments 43-73, wherein the alkylene oxide units contain from 2 to 4 carbon atoms and may be straight chained or branched.
76. The method of any one of embodiments 43-73 wherein R⁵ is linked to R¹ through an ester, thioester, or amide linkage.
77. The method of any one of embodiments 43-73 wherein R⁵ is linked to R¹ through an ester or amide linkage.
78. The method of any one of embodiments 43-71, wherein R⁵ has the formula:

   -R¹²-(N(R⁷)-R¹³)_{f}-R¹²-,

   wherein each R¹² and R¹³ are independently a 1 to 20 carbon straight-chain or branched alkyl group,
   wherein each R¹² is optionally substituted with one or more functional groups selected from the group consisting of -OH, -OR^{a}, -NR^{a}R^{b}, -CO₂H, -SO₃H,-CH₂-OR^{a}, -CH₂-O-CH₂-R^{a},-CH₂CO₂H, -CH₂SO₃H, -O-C(=O)-CH₂-CH₂-C(=O)-O- or -CH₂-NR^{a}R^{b};
   Q is -O-, -S-, and -NR^{a}- ; and
   R¹² is a 1 to 10 carbon straight-chain or branched divalent alkylene group;
   R^{a} and R^{b} are each independently hydrogen (C₁₋₆)alkyl, aryl, aryl(C₁₋₈)alkylene
   f is an integer from 2 to 150, inclusive.
79. The method of any one of embodiments 43-71 or 78, wherein R⁵ is a polyethylene imine having between 2 and 110 repeating units.
80. The method of any one of embodiments 43-71 or 78-79, wherein the polyethylene imine has units contain from 2 to 10 carbon atoms.
81. The method of any one of embodiments 43-80, wherein R⁶ is alkyl, aryl, biotin, streptavidin, sugar moieties, folic acid, amino acids or peptides.
82. The method of any one of embodiments 43-81, wherein is the peptide Arg-Gly-Asp (R-G-D) or Tyr-Ile-Gly-Ser-Arg (Y-I-GS-R).
83. The method of any one of embodiments 43-82, wherein R⁶ is biotin
84. The method of any one of embodiments 43-83, wherein the acid residue comprises from 2 to 24 carbon atoms.
85. The method of any one of embodiments 43-84, wherein the acid residue comprises from 6 to 18 carbon atoms.
86. The method of embodiment 43 wherein the acid residue comprises caprylic, capric, lauric, myristic, myristoleic, palmitic, palmitoleic, stearic, oleic, linoleic, eleostearic, arachidic, behenic, erucic acid, or a mixture thereof.
87. The method of any of embodiments 43-86, wherein the functional groups are -OH, - ORa, -NRaRb, -CO2H, -SO3H (sulfo), -CH2-OH, -CH2-ORa, -CH2-O-CH2-Ra, or -CH2-NRaRb.

### Detailed Description of the Invention

The present application discloses that amphiphilic star-like macromolecules (ASMs) and amphiphilic scorpion-like macromolecules (AScMs) are useful in certain biomedical applications, including the sequestration of LDL *in vitro*, *ex vivo* and *in vivo*, for the treatment of atherosclerosis and for the inhibition of atherosclerotic development.

### I. Definitions

"Amphiphilic scorpion-like macromolecules" is used herein interchangeably with the acronym "AScMs." "Amphiphilic star-like macromolecules" is used herein interchangeably with the acronym "ASMs."

As used herein, the phrase "low-density lipoprotein (LDL)" includes "unoxidized LDL," "weakly oxidized LDL" and "oxidized LDL." LDLs bind to proteoglycans (PGs), the major low density lipoproteins (LDL)-retentive matrix molecules within the vascular intima are proteoglycans. LDL binding to PGs modifies the LDL surface, rendering the LDL susceptible to oxidation induced by Cu²⁺ and macrophages. The oxidative modification ofLDL lowers its localized positive charge relative to native LDL, thus reducing the affinity of LDL for anionically charged PGs. The increase in the net negative charge on oxidized LDL also leads to the reduced recognition of oxidized LDL by the classical LDL receptor, and increased recognition by the scavenger receptors on macrophages in the intima. Thus, "unoxidized low-density lipoprotein" refers to a native LDL, e.g., an LDL that has the characteristics of an LDL that is recognized by a native LDL receptor. In contrast, an "oxidized LDL (ox-LDL)" is a modified LDL recognized by scavenger receptors. By the phrase "weakly oxidized low-density lipoprotein (LDL)" is meant a mildly or partially oxidized LDL. Both unoxidized and weakly oxidized LDL have relatively high localized positive charges, e.g., due to unmodified Lys and Arg residues on apolipoprotein B-100 (ApoB-100) (LDL have a single Apo B-100 molecule on their surface) as compared to oxidized LDL. See, for example, Chnari et al., Biomaterials, 26: 3749-3758 (2005).

By "sequester" is meant the separation or removal of a substance, such as LDL, for example, from a physiological sample or the blood stream ofa subject. For example, in one embodiment of the invention, ASMs or AScMs are administered to a patient and become associated with LDL in a manner that will provide a beneficial physiological effect. For example, the ASM or AScM may attach itself to LDL and cause the LDL to be eliminated from a subject, or prevent LDL from having physiological and/or pathological activity.

By "inhibition of atherosclerotic development" is meant the suppression of the development, progression and/or severity of atherosclerosis, a slowly progressive disease characterized by the accumulation of cholesterol within the arterial wall, e.g. by inhibiting, preventing or causing the regression of an atherosclerotic plaque.

As used herein, the term "targeting moiety," "targeting group," and "targeting agent" refers to groups that have an ability to direct the encapsulated active agents to a site where the activity from the active agent is desired. In the present invention, the polymers can have one or more targeting moieties. Non-limiting examples of targeting moieties include but are not limited to groups such as, for example, -CO₂H, -SO₃H (sulfo), -NH₂, or groups derived from compounds such as, for example, biotin, streptavidin, sugar moieties, folic acid, amino acids, peptides (see Jamal Temsamani, et al., PSTT, vol. 3, No. 5, May 2000, p155-162; and Mol. Pharmacol, 2000, 57, 679-686), antibodies, and antibody fragments.

A comprises a carboxy group or is absent, and when present, A may optionally be substituted with or attached to a bioactive or therapeutically active molecule. The bioactive or therapeutically active molecule may be any such molecule known to one of ordinary skill in the art including those described below. In one embodiment, the bioactive or therapeutically active molecule includes, but is not limited to, vitamin E, sulfonic acids, sulfonates, or salicylic acid; antioxidants such as β-carotene, ubiquinol-10; lipophilic agents such as probucol; compounds useful to treat atherosclerosis; and/or compounds that reduce levels, *e.g*., serum levels, of LDL.

As used herein the term "polyol" includes straight chain and branched chain aliphatic groups, as well as mono-cyclic and poly-cyclic aliphatics, which may be substituted with two or more hydroxy groups. A polyol typically 2 carbons to 20 carbons (C₁-C₂₀); preferably 3 carbons to 12 carbons (C₃-C₁₂); and more preferably 4 carbons to 10 carbons (C₄-C1₀). A polyol also typically comprises from 2 to 20 hydroxyl; preferably 2 to 12 hydroxyl; and more preferably 2 to 10 hydroxyl. A polyol also optionally may be substituted on a carbon atom with one or more, e.g. 1, 2, or 3, carboxyl (COOH), which may be used to link the polyol to a polyether in one embodiment of the compound of formula (I).

One specific polyol comprises a mono- or di-carboxyilic acid comprising 1 to 10 carbon atoms (C₁-C₁₀) and may be substituted with 1 to 10 hydroxyl. The mono-or di-carboxylic acid may be a straight chained or branched chained aliphatic, or a mono-cyclic or poly-cyclic aliphatic compound. Suitable dicarboxylic acids include mucic acid, malic acid, citromalic acid, alkylmalic acid, hydroxy derivatives of glutaric acid, and alkyl glutaric acids, tartaric acid, citric acid, hydroxy derivatives of rumadic acid, and the like. Suitable monocarboxylic acids include 2,2-(bis(hydroxymethyl)propionic acid, and N-[tris(hydroxymethyl)methyl]glycine (tricine). Other mono and di-carboxylic acids, however, are also suitable for use with this invention.

Another specific polyol comprises a "saccharide," e.g. monosaccharides, disaccharides, trisaccharides, polysaccharides and sugar alcohols, among others. The term saccharide includes glucose, sucrose, fructose, ribose, and deoxy sugars such as deoxyribose, and the like. Saccharide derivatives may be prepared by methods known to the art. Examples of suitable mono-saccharides are xylose, arabinose, and ribose. Examples of di-saccharides are maltose, lactose, and sucrose. Examples of suitable sugar-alcohols are erythritol and sorbitol. Other mono- and di-saccharide, saccharide derivatives, and sugar alcohols, however, are also suitable.

As used herein, the term polyether includes poly(alkylene oxides) of for example, 2 to 150 repeating units. Typically, the poly(alkylene oxides) comprises 50 to 110 units of the chemical formula (I), which may include the same or different residues, e.g. repeating or non-repeating units. The alkylene oxide units may comprise 2 to 20 carbon atoms, e.i. straight or branched (C₂-C₂₀) alkyl, and preferably 2 to 10 carbon atoms (C₁-C₁₀). Poly(ethylene glycol) (PEG) is one of the most prefetred. Alkoxy-, amino-, carboxy-, and sulfo-terminated poly(alkylene oxides) are preferred, with methoxy-terminated poly(alkylene oxides) being more preferred.

One preferred polyether comprises the chemical structure

R₅-(R₆-O-)ₐ-R₆-Q- (II),

wherein
R₅ comprises straight or branched (C₁-C₂₀) alkyl, -OH, -OR₇, -NH₂, -NHR₇, -NHR₇R₈, -CO₂H, -SO₃H (sulfo), -CH₂-OH, -CH₂-OR₇, -CH₂-O-CH₂-R₇, -CH₂-NH₂, -CH₂-NHR₇, -CH₂-NR₇R₈, -CH₂CO₂H, -CH₂SO₃H, or -O-C(=O)-CH₂-CH₂-C(=O)-O-;
R₆ straight or branched divalent (C₁-C₁₀) alkylene;
each R₇ and R₈ comprise, independently, straight or branched (C₁-C₆)alkylene;
Q comprises -O-, -S-, or -NR₇; and
a is an integer from 2 to 150, inclusive.

Another preferred polyether comprises methoxy terminated polyethylene glycol.

In a compound of this invention a poly(alkylene oxide) may be linked to a polyol, for example, through an ether, thioether, amine, ester, thioester, thioamide, or amide linkage. In one preferred compound the poly(alkylene oxide) may be linked to a polyol by an ester or amide linkage.

The term acyl includes fatty acid residues. As used herein, the term "fatty acid" includes fatty acids and fatty oils as conventionally defined, for example, long-chain aliphatic acids that are found in natural fats and oils. Fatty acids typically comprise 2 to 24 carbon atoms (C₁-C₁₀ fatty acids), and preferable 6 to 18 carbon atoms (C₆-C₁₈ fatty acids). The term "fatty acid" encompasses compounds possessing a straight or branched aliphatic chain and an acid group, such as a carboxylate, sulfonate, phosphate, phosphonate, and the like. The "fatty acid" compounds are capable of "esterifying" or forming a similar chemical linkage with hydroxy groups on the polyol. Examples of suitable fatty acids include caprylic, capric, lauric, myristic, myristoleic, palmitic, palmitoleic, stearic, oleic, linoleic, eleostearic, arachidic, behenic, erucic, and like acids. Fatty acids may be derived from suitable naturally occurring or synthetic fatty acids or oils, may be saturated or unsaturated, and optionally may include positional and/or geometric isomers. Many fatty acids or oils are commercially available or may be readily prepared or isolated using procedures known to those skilled in the art.

The nature of the "linker" is not critical provided it does not interfere with the desired function of the compound or conjugate. For example, the linker can include a straight or branched carbon chain having from one to 20 carbon atoms; the carbon chain can optionally be saturated or unsaturated and can optionally be interrupted with one or more heteroatoms (e.g. oxygen, sulfur, or nitrogen). In one embodiment, the linker has from 2 to 10 carbon atoms.

### II. Polymer Micelles

Polymeric micelles that can be used to sequester low-density lipoproteins (LDLs) include micelles as disclosed in International application Serial No. PCT/US03/17902; International application Serial No. PCr/US02/21923 and International application Serial No. PCTmS00/10500.

Accordingly, a polymeric micelle that can be used to sequester low-density lipoproteins (LDLs) includes a compound of formula (**I**):

A-X-Y-Z-R₁ (**I**)

wherein **A** is a carboxy group or is absent; X is a polyol, Y is -C(=O)-,-C(=S)-, or is absent; **Z** is O, S or NH; and R1 is a polyether, wherein one or more hydroxy groups of the polyol are acylated with a fatty acid residue.

In another embodiment of the invention, a polymeric micelle that can be used to sequester low-density lipoproteins (LDLs) includes a polymer having a structure selected from the group consisting of:

R (-O-R₁), and R(-NH-R₁)]ₓ

wherein R(-O-)ₓ is a polyol moiety and R (-NH)ₓ is a polyamine moiety, with x being between 2 and 10, inclusive, and each R₁ independently has the structure: 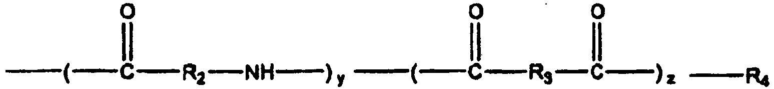 wherein 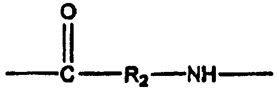 is a divalent amino acid moiety with R₂ being a covalent bond or having from 1 to 8 carbon atoms, and y and z are between 0 and 10, inclusive, provided that y and z are not both 0;
wherein 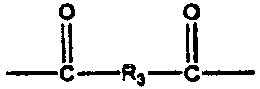 is a divalent dicarboxylic acid moiety in which R₃ is an alkylene or cycloalkylene group containing from 1 to 15 carbon atoms, substituted with a total of from 1 to 10 hydroxyl groups, with at least a portion of the hydroxyl groups being acylated with from 3 to 24 carbon atom carboxylic acids; and wherein R₄ is a poly (alkylene oxide) having the structure:

R₅ - (R₆-O-) a - R₆-Q-

with R₅ being selected from the group consisting of 1 to 40 carbon atom alkyl groups, -OH, -OR₇, -NH₂, -NHR₇, -NHR₇R₈, -C-OH, -C-OR₇, -C-O-C-R₇, -C-NH₂, -C-NHR₇ and -C-NR₇R₈; R₆, R₇ AND R₈ being independently selected from the group consisting of 2 to 40 carbon atom, straight-chain or branched alkylene groups; Q being a divalent linkage moiety; and a being between 2 and 110, inclusive. In one embodiment, when y is 0 and R is a 1, 1, 1-tris (hydroxyphenyl) ethane moiety, the divalent dicarboxylic acid moiety is not a mucic acid moiety.

In another embodiment of the invention, a polymeric micelle that can be used to sequester low-density lipoproteins (LDLs) includes a compound having formula (II):

R¹-((R²)ₐ-(R³)_{b}-(R⁴)_{c}-(R⁵)_{d}-(R⁶)_{c})ₙ (**II**)

wherein R¹ is a core comprising a polyol or polyacid; each R² independently is a divalent or polyvalent group having the formula X¹-R⁸-(X^{1a})₈-, wherein X' and X^{1a} are independently -C(=O)-, -C(=S)-, -O-, -S-, -N(R⁷)- or absent, and each R⁸ is independently -(C₁₋₈)alkylene-, branched -(C₁₋₈)alkylene- or -(C₆₋₁₀)aryl-; a is 0 or an integer from 1 to 10; and g is an integer from 1 to 6; each R³ independently is a divalent dicarboxylic acid moiety having the formula -C(=O)-R⁹-C(=O)-, wherein R⁹ is an alkylene or cycloalkylene group containing from 1 to 15 carbon atoms, substituted with a total of from 1 to 10 hydroxy groups, wherein one or more of the hydroxy groups of the dicarboxylic acid are acylated with an acid residue; and b is an integer from 1 to 10;
each R⁴ independently is a divalent or polyvalent group having the formula -X²-R¹⁰-(X^{2a})ₕ-, wherein X² is -C(=O)-, -C(=S)-, -O-, -S-, -N(R⁷)- or absent; X^{2a} is -C(=O)-, -C(=S)-, -O-, -S-, or -N(R⁷)- and R¹⁰ is -(C₁₋₈)alkylene-, branched -(C₁₋₈)alkylene- or -(C₆₋₁₀)aryl-; and c is 0 or an integer from 1 to 10; and h is an integer from 1 to 6;
each R⁵ independently is a group having the formula:

   -R¹²-(R¹¹)_{f}-R¹²-X³-

   wherein R¹¹ is a sugar moiety; or a poly(alkylene oxide) or poly(alkylene imine) group having the formula -(-X⁴-R¹³)-; wherein R¹³ is -(C₂₋₄₀)alkylene- or branched -(C₃₋₄₀)alkylene-; wherein each X³ is independently -C(=O)-, -C(=S)-, -O-, -S-, -N(R⁷)- or absent; each X⁴ is independently -O-, or -N(R⁷)-; and f is an integer from 2 to 150; and d is from 1 to 6;
   each R¹² is independently a bond, -(C₁₋₄₀)alkylene- or branched -(C₁₋₄₀)alkylene- groups, wherein each R¹² is optionally substituted with one or more (e.g., 1, 2, or 3) functional group; and X⁴ is -0-, -S-, or -N(R⁷)-.

In certain embodiments, four criteria may be employed in the design of amphiphilic scorpion-like macromolecules. First, a tunable hydrophilic-lipophilic balance (HLB) is desired to match the AScMs with the hydrophobicity of a candidate drug to optimize drug-carrier interactions. Second, polymer systems themselves should not cause any undesirable biological complications, such as toxicity and immunogenicity. See, e.g. Moghimi, S. M., Adv. Drug Delivery Rev. 1995, 17, 1. Third, the polymers are preferably biodegradable and easily excretable by living systems. Fourth, the inclusion of biological functionality significantly aids in the selective biomedical applications.

A series of AscM macromolecules that meet these four criteria were prepared. See, Tian et al., Macromolecules, 2004, 37, 538-543. The exemplary macromolecules were synthesized employing mucic acid, monohydroxy-poly(ethylene glycol) (PEG), and acyl chlorides. These particular AScMs are referred to as MₓP_{y}, in which M denotes mucic acid; x denotes the total carbon number of each acyl chain; P denotes PEG, and y refers to molecular weight of the PEG in thousands. Mucic acid has four hydroxyl groups that are acylated by a series of acyl chlorides of varying chain lengths. The alkyl chain lengths may be tuned to custom tailor the structure and properties of the resulting polymers. A multi-branched hydrophobic domain is proposed to be more efficient in self-assembly in aqueous media than a single hydrophobic block. See, e.g. Kreig et al., J. Chem. Phys. 2001, 115, 6243-6251; Gitsov et al., J. Polym. Sci., Part A: Polym. Chem. 2000, 38, 2711-2727. Mucic acid's two carboxylic acids may be selectively activated and conjugated to bioactive molecules. Mucic acid is a naturally occurring compound that enhances the biocompatibility of the AScMs polymers. See, e.g. Schmalenberg et al., Biomacromolecules 2001, 2,851-855. Finally, PEG was chosen for its well-known biological significance. When provided with a PEG-based shell, microparticles, such as micelles, liposomes, and nanoparticles, may avoid the adsorption of proteins and adhesion of cells in biological media. See, e.g. Otsuka et al., Curr. Opin. Colloid Interface Sci. 2001, 6, 3-10.

### III. Dosages and Routes of Administration of the Polymer Micelles

The amphiphilic star-like macromolecules (ASMs) and amphiphilic scorpion-like macromolecules (AScMs) may be formulated as pharmaceutical compositions, and may be administered to a mammalian host, such as a human patient, in a variety of forms adapted to the chosen route of administration, *i.e*., orally orparenterally, by intravenous, intramuscular, topical, subcutaneous, or other routes. Thus, the AScMs of the invention may be systemically administered, *e.g*., orally, in combination with a pharmaceutically acceptable vehicle such as an inert diluent. They may be incorporated directly with the food of the patient's diet. For oral therapeutic administration. the ASMs or AScMs of the invention may be used in the form of elixirs, syrups, and the like.

The compositions may also contain a sweetening agent such as sucrose, fructose, lactose or aspartame or a flavoring agent such as peppermint, oil of wintergreen, or cherry flavoring may be added. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the ASMs or AScMs of the invention may be incorporated into sustained-release preparations and devices.

The ASMs or AScMs of the invention may also be administered intravenously or intraperitoneally by infusion or injection, among many other routes. Solutions of the ASMs or AScMs may be prepared, for example, in water. However, other solvents may also be employed. Under ordinary conditions of storage and use, these preparations may contain a preservative to prevent the growth of microorganism, and other formulation ingredients as is known in the art.

The pharmaceutical dosage forms suitable for injection or infusion should be preferably sterile, fluid and stable under the conditions of manufacture and storage. The prevention of the action of microorganisms may be brought about by various antibacterial and antifungal agents, for example, parabens, chlorobutanol, phenol, sorbic acid, thimerosal, and the like. Others are also suitable. In many cases, it may be preferable to include isotonic agents, for example, sugars, buffers or sodium chloride.

Sterile injectable solutions may be prepared by incorporating the ASMs or AScMs of the invention in the required amount into an appropriate solvent or medium with various other ingredients, *e.g*., those enumerated above, as needed, which may be followed by sterilization.

The dose and method of administration will vary from animal to animal and be dependent upon such factors as the type of animal being treated, its sex, weight, diet, concurrent medication, overall clinical condition, the particular therapeutic agent employed, the specific use for which the agent is employed, and other factors which those skilled in the relevant field will recognize.

Therapeutically effective dosages may be determined by either *in vitro, ex* vivo, or *in vivo* methods, in accordance with the intended application. For each particular dosage form of the present invention, individual determinations may be made by an artisan to determine the optimal dosage required. The range of therapeutically effective dosages will naturally be influenced by the route of administration, the therapeutic or diagnostic objective, and the condition of the patient. The determination of effective dosage levels, that is the dosage level necessary to achieve a desired result, will be within the ambit of one skilled in the art. Typically, applications of an agent such as the one of this invention are commenced at low dosage levels, with dosage levels being increased until the desired effect is achieved.

A typical dosage might range from 0.001 mg to 1,000 mg of agent per kg of animal weight (mg/kg). Preferred dosages range from 0.01 mg/kg to 100 mg/kg, and more preferably from 0.10 mg/kg to 20 mg/kg. Advantageously, the dosage forms of this invention may be administered, for example, as a single dose, or several times per day, and other dosage regimens may also be useful. The period of time during which the present product may be administered may vary with the intended application. For acute instances, the administration or application may be conducted for short periods of time, *e.g*. a few days to one or more weeks or months. For chronic problems, the administration or application may be conducted for even longer periods of time, up to one or more years, or for life, with appropriate monitoring.

The following non-limiting examples set forth hereinbelow illustrate certain aspects of the invention, All parts and percentages are by weight unless otherwise noted and all temperatures are in degrees Celsius.

All PEG's were obtained from Shearwater Polymers (Birmingham, AL) and used without further purification. All other chemicals were obtained from Aldrich (Milwaukee, WI),and used without further purification. Analytical grade solvents were used for all the reactions. Methylene chloride, tetrahydrofuran (THF), triethylamine (TEA) and dimethylsulfoxide (DMSO) were distilled. 4-(dimethylamino) pyridinium p-toluenesulfonate (DPTS) was prepared as described by J.S. Moore, S.L Stupp Macromolecules 1990, 23, 65. ¹H-NMR and spectra were recorded on a Varian 200 MHz or 400 MHz spectrometer. Samples (∼5-10 mg/ml) were dissolved in CDCl₃ or THF-d₄, with the solvent used as an internal reference. IR spectra were recorded on a Mattson Series spectrophotometer by solvent casting samples onto a KBr pellet. Thermal analysis data were determined on a Perkin-Elmer Pyris 1 DSC system, samples (∼10 mg) were heated under dry nitrogen gas. Data were collected at heating and cooling rates of 5 °C/min. Gel permeation chromatography (GPC) was performed on a Perkin-Elmer Series 200 LC system. Dynamic laser scattering (DSL) measurements were carried on NICOMP particle sizing systems.

### IV. Methods for Treating Diseases Associated With Pathological Cells

There are certain problems related to a lack of specificity in cancer and other therapeutics. The lack of specificity results in the administration of larger than needed doses of compounds. The larger than needed doses of compounds administered to the patient cause unwanted or unpleasant side effects to the patient. Side effects may include, but are not limited to, nausea, emesis, loss of hair, fungal infections, bacterial infections, anemia, leucopenia, thrombocytopenia, neutropenia, enteritis, mucositis, and pain. What is needed are methods to specifically target therapeutic agents to preselected sites, tissues, or cells in the body.

Amphiphilic macromolecules are a known class of compounds that typically have both polar and non-polar regions present in the macromolecule. Amphiphilic macromolecules useful according to the methods of the present invention typically have diameters in the range of 5 to 250 nm.

It has been unexpectedly discovered that when therapeutic agents are associated with amphiphilic macromolecules, there is an increase in uptake of the agents in certain types of pathological cells (e.g. cancer cells and cells associated with the inflammatory process).

PGT/US02/21923, PCT/US00/10500, and PCT/US03/17902 report the use of amphiphilic macromolecule encapsulated therapeutic agents. When a therapeutic agent is associated with these molecules, it has unexpectedly been discovered that the uptake of the therapeutic agent can be improved in certain specific types of cells. For example, cancer cells, tumor cells, and cells related to the inflammatory process

Therapeutic agents useful in the present invention include, but are not limited to, anti-cancer, anti-tumor, anti-metastasis and anti-inflammatory agents.

In one embodiment of the invention, the anti-cancer compounds useful in the present invention, include anti-neoplastic compounds, for example, 6-azauridine, 6-diazo-5-oxo-L-norleucine, 6-mercaptopurine, aclacinomycin(s), ancitabine, anthramycin, azacitadine, azaserine, bleomycin(s), capecitabine, carubicin, carzinophillin A, chlorozotocin, chromomycin(s), cladribine, cytarabine, daunorubicin, denopterin, docetaxel, doxifluridine, doxorubicin, edatrexate, eflomithine, elliptinium, enocitabine, epirubicin, etoposide, floxuridine, fludarabine, gemcitabine, idarubicin, mannomustine, melphalan, menogaril, methotrexate, mitobronitol, mitolactol, mitomycin C, mitoxantrone, mopidamol, mycophenolic acid, nogalamycin, olivomycin(s), paclitaxel, pentostatin, peplomycin, pirarubicin, piritrexim, plicamycin, podophyllinic acid 2-ethylhydrazine, prednimustine, procarbazine, pteropterin, puromycin, ranimustine, streptonigrin, streptozocin, teniposide, thiamiprine, thioguanine, Tomudex® (N-[[5-[[(1,4-Dihydro-2-methyl-4-oxo-6-quinazolinyl)methyl]methylamino]-2-thienyl]carbonyl]-L-glutamic acid), toptecan, trimetrexate, tubercidin, ubenimex, vinblastine, vindesine, vinorelbine, zorubicin and the like.

In another embodiment of the invention, the anti-neoplastic compounds suitable for use in the present invention include, but are not limited to 6-diazo-5-oxo-L-norleucine, azaserine, carzinophillin A, denopterin, edatrexate, eflornithine, melphalan, methotrexate, mycophenolic acid, podophyllinic acid 2-ethylhydrazide, pteropterin, streptonigrin, Tornudex® (N-((5-(((1,4-Dihydro-2-methyl-4-oxo-6-quinazolinyl)methyl)methylamino)-2-thienyl)carbonyl)-L-glutamic acid), ubenimex, and the like.

In another embodiment of the present invention, non-steroidal anti-inflammatory (NSAID) compounds suitable for use in the present invention include, but are not limited to 3-amino-4-hydroxybutyric acid, aceclofenac, alminoprofen, bromfenac, bumadizon, carprofen, diclofenac, diflunisal, enfenamic acid, etodolac, fendosal, flufenamic acid, gentisic acid, meclofenamic acid, mefenamic acid, mesalamine, niflumic acid, olsalazine oxaceprol, S-adenosylmethionine, salicylic acid, salsalate, sulfasalazine, tolfeasmic acid, and the like.

In another embodiment of the invention, the diameter of the macromolecule is less than 250 nm. In another embodiment of the invention, the diameter of the macromolecule is less than 150 nm. In another embodiment of the invention, the diameter of the macromolecule is less than 100 nm. In another embodiment of the invention, the diameter of the macromolecule is less than 80 nm. In another embodiment of the invention, the diameter of the macromolecule is less than 70 nm. In another embodiment of the invention, the diameter of the macromolecule is less than 60 nm. In another embodiment of the invention, the diameter of the macromolecule is less than 50 nm. In another embodiment of the invention, the diameter of the macromolecule is less than 40 nm. In another embodiment of the invention, the diameter of the macromolecule is less than 30 nm. In another embodiment of the invention, the diameter of the macromolecule is less than 25 nm. In another embodiment of the invention, the diameter of the macromolecule is less than 20 nm.

In another embodiment of the invention, the diameter of the macromolecule is greater than 5 nm. In another embodiment of the invention, the diameter of the macromolecule is greater than 10 nm. In another embodiment of the invention, the diameter of the macromolecule is greater than 15 nm. In another embodiment of the invention, the diameter of the macromolecule is greater than 20 nm. In another embodiment of the invention, the diameter of the macromolecule is greater than 25 nm. In another embodiment of the invention, the diameter of the macromolecule is greater than 30 nm. In another embodiment of the invention, the diameter of the macromolecule is greater than 35 nm. In another embodiment of the invention, the diameter of the macromolecule is greater than 40 nm. In another embodiment of the invention, the diameter of the macromolecule is greater than 45 nm. In another embodiment of the invention, the diameter of the macromolecule is greater than 50 nm. In another embodiment of the invention, the diameter of the macromolecule is greater than 55 nm.

### EXAMPLES

### Example 1: Synthesis of AScMs

AScMs may be prepared using techniques similar to those described in PCT/US03/1T902 (WO 03/103594). For example, as illustrated below, a representative compound of formula (I) may be prepared. 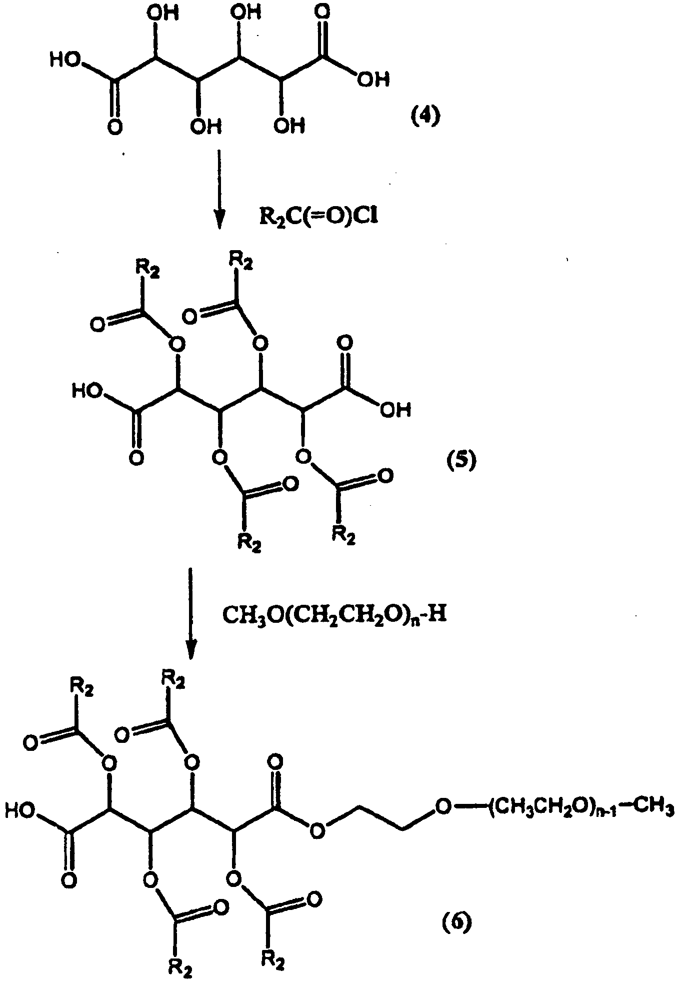 A polyol (**4**) is acylated by reaction with a stoichiometric excess of a fatty acid chloride to provide acylated polyol (**5**). Suitable conditions for such an acylation reaction are well known. For example, the reaction may be carried out in the presence of a catalyst, such as ZnCl₂, with heating. Suitable acylation conditions are described in PCT/US03/17902. Coupling of (**5**) with a polyether, for example through an ester linkage, using a suitable coupling agent provides the compound of formula (**I**) (**6**).

In particular, compound (**5**), wherein R₂ = ethyl was synthesized as follows: To a neat mixture of mucic acid (4.2 g, 20 mmol) and propionyl chloride (18 ml, 200 mmol) was added ZnCl₂ (0.28 g, 2.0 mmol). The reaction mixture was heated at reflux temperature for three hours. After cooling, diethyl ether (20 ml) was added to the reaction mixture and the solution poured onto ice chips (approximately 100 g) with stirring. Additional diethyl ether (80 ml) was added to the mixture and stirring continued for 30 minutes more. The ether portion was separated, washed with water to a neutral pH, dried over anhydrous Na₂SO₄ and evaporated to dryness. The crude product was purified by recrystallization from a cosolvent system of diethyl ether and methylene chloride, collected by vacuum filtration, washed by ice cold methylene chloride and dried at 105°C (12 hours) to 15 constant weight. The title compound was obtained as a white solid having a Tₘ of 196°C, 56% yield.

### Example 2: Synthesis of Mucic Acid-Acylated PEG AScMs

AScMs were synthesized from mucic acid, monohydroxy-poly( ethylene glycol) (PEG) and acyl chlorides. The four hydroxyl groups of mucic acid were acylated using acyl chlorides with varying chain lengths. Zinc chloride was added as catalyst, the reactions were performed using acyl chlorides as solvent at 90°C. The acylated derivatives of mucic acid were coupled onto the PEG with 1,3-dicyclohexylcarbodiimide (DCC) as coupling agent and 4-(dimethylamino) pyridinium p-toluenesulfonate (DPTS) as catalyst to yield the amphiphilic polymers.

Similar to conventional amphiphilic diblock copolymers (Gao and Eisenberg, Macromolecules 1993, 26, 7353-7360; Tuzar and Kratochvil, Surf. Colloid Sci. 1993, 15, 1-83; and Cammas et al., MacromoL Chem. Phys. 1995,196,1899-1905), AScMs systems have a hydrophilic block (*i.e*., PEG) that is modified by a hydrophobic portion (*i.e*., acylated mucic acid derivatives). Unlike amphiphilic diblock copolymers, the hydrophobic component of the MₓPy materials is multi-branched. The extremely hydrophobic, multi-branched domain contributes to forming a stable aggregation in an aqueous system, which is ultimately a function of HLB. At the concentrations above critical micelle concentration (CMC), AScMs form micelles with the interior hydrophobic core and the hydrophilic chains extending outside. The CMC values for the AScMs polymers range between 10⁻⁵ M and 10⁻⁷ M. The low CMC values illustrate the effectiveness of multi-branched structure for stabilizing micellar aggregates. With such low CMC values, AScMs may form a highly stable micellar aggregates with low rates of dissociation in vivo (Allen et al., Colloids Surf., B: Biointerfaces 1999, 16,3-27). By dynamic light scattering (DLS) analyses, all micellar aggregates have unimodal size distribution at around 10 - 20 nm within ± 2 nm derivations (data not shown). Additionally, transmission electron microscopy studies (TEM) confirmed such nanoscale particles (data not shown). The sizes were independent of both PEG and alkyl chain lengths. Above the critical micelle concentrations, the aggregations are quite stable upon dilution.

Both unoxidized (native) and weakly oxidized LDL were sequestered by low concentrations (10⁻⁴ M) of carboxylate-terminated nanocarriers. Under the oxidation conditions used, both unoxidized and mildly oxidized LDL have localized (ApoB-100) positive charge and thus showed low levels of electrophoretic mobility (REM of 1 for unoxidized LDL; 1.5 for weakly oxidized LDL). As mild oxidative modification involves mostly lipid oxidation and doesn't alter the positively charged amino acids of the Apo B-100, the native and mildly (weakly) oxidized LDL had relatively high localized positive charge (due to unmodified Lys and Arg residues on the ApoB-100), which induced electrostatic association with the anionic nanocarriers. Similar trends have been reported in the literature regarding LDL association with anionically charged groups of specific proteoglycans *in vitro.* Mild oxidation of LDL increases LDL association to heparin sulfate (HSPG), whereas higher LDL oxidation decreases the association rate and binding affinity to levels below those for native LDL binding.

For drug delivery applications, these stable, narrow-distributed nanocarriers are expected to show extravasation efficacy for solid tumor tissues (Moghimi et al.; J. Pharm. Rev. 2001, 53, 283-318) and evade reticuloendothelial system up-take (Papisov, Adv. Drug Delivery Rev. 1995, 16, 127-139).

### Example 3: Sequestration of LDL

In addition to drug delivery applications, AScMs are useful as mobile nanoscale substrates for the sequestration of unoxidized and/or weakly oxidized low-density lipoproteins (LDL) for the systematic inhibition of atherosclerotic development. See, e.g. Wiliams and Tabas, Arteriosclerosis, Thrombosis & Vascular Biology, 1993,15: 551-561. These AScMs as nanocarriers may compete with proteoglycans for LDL binding; to capture LDL before it gets extensively oxidized and transport to cells in its least atherogenic form for controlled cellular uptake and metabolism. Interactions between LDL and the anionic nanocarriers of the present AScMs were confirmed using both dynamic light scattering and transmission electron microscopy. LDL-AScM complexes of about 60-90 nm in size were detected and visualized, which demonstrated that LDL may be sequestered, e.g. due to electrostatic interactions between the anionic AScMs and positively charged amino acid residues on the LDL particle. See, e.g. Camejo et al., Atherosclerosis Supplements, 2002, 3: 3-9. The presence of the AScM hydrcphobie core close to the carboxylate functionality may further chance LDL binding through hydrophobic-hydrophobic interactions.

### Example 4: Cellular internalization of amphiphilic-scoripion like macromolecules (AScMs): implication for drug delivery

*Methods* Amphiphilic scorpion-like macromolecules (AScMs) are block copolymers that form micelles at concentrations above their CMC (5 x 10⁻⁷ M). The uptake of AScMs was investigated in human umbilical vein endothelial cells (HUVECs) by monitoring cellular accumulation of the fluorescent dyes (R110, R123 and LY, respectively). Briefly, fluorescent dyes were added to AScMs solutions (0.0001 - 1 mM) and incubated at 37 °C for at least 6 hours prior to cell incubation. After determination of R110, R123 and LY partitioning into the AScMs micelles, dye-loaded AScMs were incubated with HUVEC. Uptake of dye-loaded AScMs was investigated as a function of time (15, 30, 60, 90 and 120 minutes) and AScMs concentration (0.0001 - 1 mM). Furthermore, intracellular distribution and localization of dye-loaded AScMs in HUVEC was investigated using confocal microscope.

*Results* At concentrations below the CMC, a significant increase in dye (*i.e*, R110, R123 and LY) accumulation in the HUVEC monolayers was observed; whereas at concentrations above the CMC, a significant decrease in dye accumulation was noted. The decreased accumulation of R110, R123 and LY in HUVEC monolayers was inversely correlated with the extent of their partitioning into AScMs. Uptake of R110, R123 and LY-loaded micelles was also time-dependent. The uptake was seen as early as 15 minutes, and gradually increased with incubation time. Furthermore, confocal microscopy studies confirmed increased fluorescence activity in the cells with increase in the incubation time.

*Conclusions* Cellular uptake and intracellular retention of amphiphilic scorpion-like macromolecules (AScMs) was demonstrated *in vitro* using human umbilical vein endothelial cells (HUVEC). AScMs were shown to be rapidly internalized into the HUVEC. Cellular uptake was shown to be time- and AScM concentration-dependent, and the micelles were mainly localized in the cytoplasm. Thus, AScMs can be used to localize therapeutic compounds into cells, for example, to achieve a sustained therapeutic effect.

All publications, patents, and patent documents, particularly all relevant sections of the documents mentioned in this patent are incorporated by reference herein, as though individually incorporated by reference. The invention has been described with reference to various specific and preferred embodiments and techniques. However, it should be understood that many variations and modifications may be made while remaining within the spirit and scope of the invention.

## Claims

1. Use of a compound of chemical formula (I)
A-X-Y-Z-R₁ (I)
wherein A comprises a carboxy group or is absent;
X comprises a polyol, wherein one or more polyol hydroxyls are substituted by acyl;
Y comprises -C(=O)-, -C(=S)-, or is absent;
Z comprises O, S or NH; and
R₁ comprises a polyether;
for the preparation of a medicament for inhibiting or reducing atherosclerosis or atherosclerotic development in an animal.

2. The use of claim 1, wherein in the polyol acyl comprises a fatty acid(s).

3. The use of claim 1 or 2, wherein the polyol comprises a (C₂-C₂₀) alkyl polyol.

4. The use of any one of claims 1 to 3, wherein the polyol comprises about 2 to about 20 hydroxyl groups.

5. The use of any one of claims 1 to 4, wherein the polyol is substituted with one or more acyl.

6. The use of any one of claims 1 to 5, wherein the polyol comprises a mono- or dicarboxylic (C₂-C₂₀) alkyl polyol substituted with about 1 to about 10 hydroxyl(s).

7. The use of any one of claims 1 to 6, wherein the polyol comprises one or more of mucic acid, malic acid, citromalic acid, alkylmalic acid, hydroxy glutaric acid derivatives, alkyl glutaric acids, tartaric acid, or citric acid.

8. The use of any one of claims 1 to 7, wherein the polyol comprises one or more of 2,2-(bis(hydroxymethyl)propionic acid, tricine, or a saccharide.

9. The use of any one of claims 1 to 8, wherein the polyether comprises about 2 to about 150 alkylene oxide units.

10. The use of any one of claims 1 to 9, wherein each alkylene oxide unit comprises straight or branched (C₂-C₄) alkylene oxide.

11. The use of any one of claims 1 to 10, wherein the polyether comprises an alkoxy-terminal group.

12. The use of any one of claims 1 to 11, wherein the polyether is linked to the polyol through a linker comprising ester, thioester, or amide.

13. The use of any one of claims 1 to 12, wherein the polyether comprises the chemical formula
R₅-(R₆-O-)ₐ-R₆-Q- (II),
wherein
R₅ comprises straight or branched (C₁-C₂₀) alkyl, -OH, -OR₇, -NH₂, -NHR₇, -NHR₇R₈, - CO₂H, -SO₃H (sulfo), -CH₂-OH, -CH₂-OR₇, -CH₂-O-CH₂-R₇, -CH₂-NH₂, -CH₂-NHR₇, -CH₂-NR₇R₈, -CH₂CO₂H, -CH₂SO₃H, or -O-C(=O)-CH₂-CH₂-C(=O)-O-;
R₆ comprises straight or branched divalent (C₂-C₁₀) alkylene;
each R₇ and R₈ comprises, independently, straight or branched (C₁-C₆) alkylene;
Q comprises -O-, -S-, or -NR₇; and
a comprises an integer of about 2 to about 110, inclusive.

14. The use of any one of claims 1 to 13, wherein the polyether comprises a polyethylene glycol comprising a methoxy terminal group.

15. The use of any one of claims 2 to 14, wherein the fatty acid(s) comprise(s) (C₂-C₂₄) fatty acid(s).

16. The use of any one of claims 2 to 15, wherein the fatty acid(s) comprise(s) one or more of caprylic, capric, lauric, myristic, myristoleic, palmitic, palmitoleic, stearic, oleic, linoleic, arachidic, behenic, or erucic acid.

17. The use of any one of claims 1 to 16, wherein the compound of formula (I) has the chemical structure 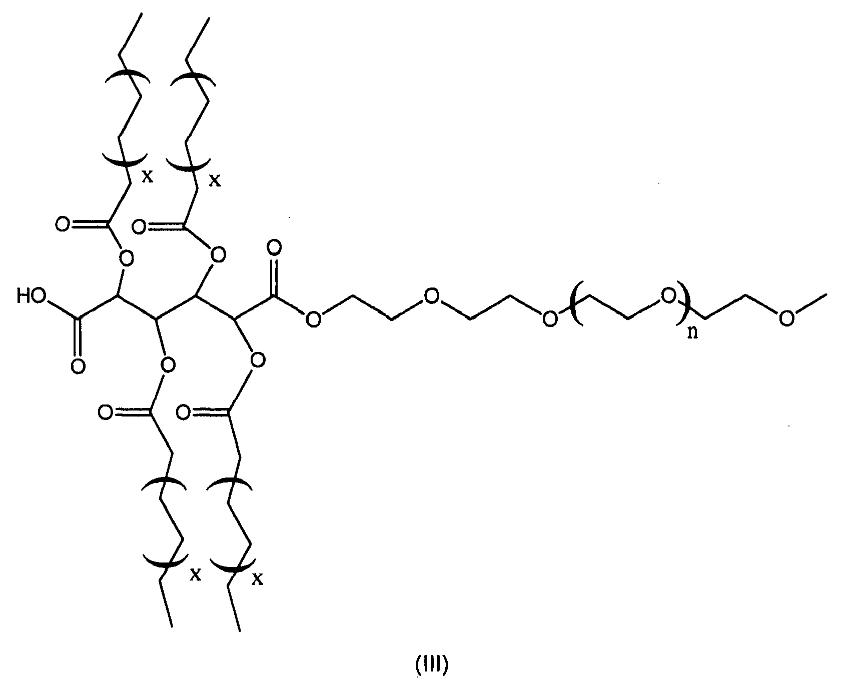 wherein each x comprises, independently, 1, 2, 3, or 4; and n is about 36.

18. The use of any one of claims 1 to 17, wherein the compound of formula (I) is provided in the form of a nanoparticulate formulation.

19. The use of claim 1, wherein the animal is a human.

20. Compound of chemical formula (I)
A-X-Y-Z-R₁ (I)
wherein A comprises a carboxy group or is absent;
X comprises a polyol, wherein one or more polyol hydroxyls are substituted by acyl;
Y comprises -C(=O)-, -C(=S)-, or is absent;
Z comprises O, S or NH; and
R₁ comprises a polyether;
for use for inhibiting or reducing atherosclerosis or atherosclerotic development in an animal.

## Patentansprüche

1. Verwendung einer Verbindung der chemischen Formel (I)
A-X-Y-Z-R₁ (I)
wobei A eine Carboxygruppe umfasst oder nicht vorhanden ist;
X ein Polyol umfasst, wobei ein oder mehrere Polyolhydroxyle durch Acyl substituiert sind;
Y -C(=O)-, -C(=S)- umfasst oder nicht vorhanden ist;
Z O, S oder NH umfasst; und
R₁ einen Polyether umfasst;
für die Herstellung eines Medikaments zum Hemmen oder Reduzieren von Atherosklerose oder einer atherosklerotischen Entwicklung in einem Tier.

2. Verwendung nach Anspruch 1, wobei in dem Polyol Acyl eine oder mehrere Fettsäure(n) umfasst.

3. Verwendung nach Anspruch 1 oder 2, wobei das Polyol ein (C₂-C₂₀)-Alkylpolyol umfasst.

4. Verwendung nach einem der Ansprüche 1 bis 3, wobei das Polyol ungefähr 2 bis ungefähr 20 Hydroxylgruppen umfasst.

5. Verwendung nach einem der Ansprüche 1 bis 4, wobei das Polyol mit einem oder mehreren Acyl substituiert ist.

6. Verwendung nach einem der Ansprüche 1 bis 5, wobei das Polyol ein Mono- oder Dicarbonsäure(C₂-C₂₀)-alkylpolyol substituiert mit ungefähr 1 bis ungefähr 10 Hydroxyl(en) umfasst.

7. Verwendung nach einem der Ansprüche 1 bis 6, wobei das Polyol eines oder mehrere von Schleimsäure, Äpfelsäure, Citroäpfelsäure (citromalic acid), Alkyläpfelsäure, Hydroxyglutarsäurederivaten, Alkylglutarsäuren, Weinsäure oder Citronensäure umfasst.

8. Verwendung nach einem der Ansprüche 1 bis 7, wobei das Polyol eines oder mehrere von 2,2-(Bis(hydroxymethyl)propionsäure, Tricin oder einem Saccharid umfasst.

9. Verwendung nach einem der Ansprüche 1 bis 8, wobei der Polyether ungefähr 2 bis ungefähr 150 Alkylenoxideinheiten umfasst.

10. Verwendung nach einem der Ansprüche 1 bis 9, wobei jede Alkylenoxideinheit geradkettiges oder verzweigtes (C₂-C₄)-Alkylenoxid umfasst.

11. Verwendung nach einem der Ansprüche 1 bis 10, wobei der Polyether eine Alkoxy-Endgruppe umfasst.

12. Verwendung nach einem der Ansprüche 1 bis 11, wobei der Polyether über ein Ester, Thioester oder Amid umfassendes Bindeglied mit dem Polyol verbunden ist.

13. Verwendung nach einem der Ansprüche 1 bis 12, wobei der Polyether die chemische Formel
R₅-(R₆-O-)ₐ-R₆-Q- (II)
umfasst, wobei
R₅ ein geradkettiges oder verzweigtes (C₁-C₂₀)-Alkyl, -OH, -OR₇, -NH₂, -NHR₇, -NHR₇R₈, -CO₂H, -SO₃H (Sulfo), -CH₂-OH, -CH₂-OR₇, -CH₂-O-CH₂-R₇, -CH₂-NH₂, -CH₂-NHR₇, -CH₂-NR₇R₈, -CH₂CO₂H, -CH₂SO₃H, oder -O-C(=O)-CH₂-CH₂-C(=O)-O- umfasst;
R₆ geradkettiges oder verzweigtes zweiwertiges (C₂-C₁₀)-Alkylen umfasst;
jedes R₇ und R₈ unabhängig voneinander geradkettiges oder verzweigtes (C₁-C₆)-Alkylen umfasst;
Q -O-, -S- oder -NR₇ umfasst; und
a eine ganze Zahl von einschließlich ungefähr 2 bis einschließlich ungefähr 110 umfasst.

14. Verwendung nach einem der Ansprüche 1 bis 13, wobei der Polyether ein Polyethylenglycol umfasst, das eine Methoxy-Endgruppe umfasst.

15. Verwendung nach einem der Ansprüche 2 bis 14, wobei die Fettsäure(n) (C₂-C₂₄)-Fettsäure(n) umfasst (umfassen).

16. Verwendung nach einem der Ansprüche 2 bis 15, wobei die Fettsäure(n) eine oder mehrere von Caprylsäure, Caprinsäure, Laurinsäure, Myristinsäure, Myristoleinsäure, Palmitinsäure, Palmitoleinsäure, Stearinsäure, Ölsäure, Linolsäure, Arachinsäure, Behensäure oder Erucasäure umfasst (umfassen).

17. Verwendung nach einem der Ansprüche 1 bis 16, wobei die Verbindung der Formel (I) die chemische Struktur aufweist 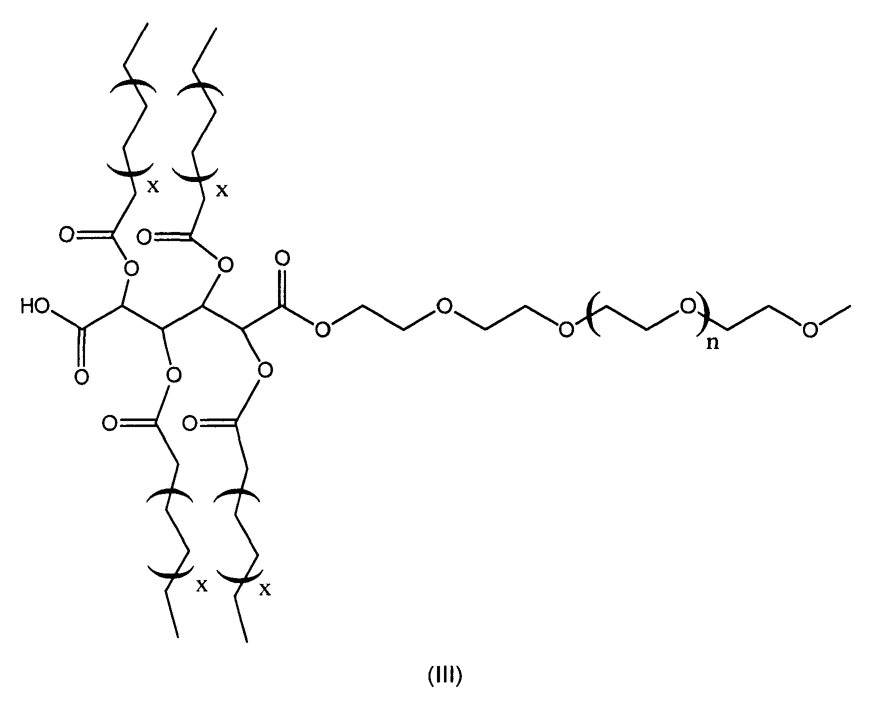 wobei jedes x unabhängig voneinander 1, 2, 3 oder 4 umfasst; und n ungefähr 36 ist.

18. Verwendung nach einem der Ansprüche 1 bis 17, wobei die Verbindung der Formel (I) in Form einer nanopartikulären Formulierung bereitgestellt wird.

19. Verwendung nach Anspruch 1, wobei das Tier ein Mensch ist.

20. Verbindung der chemischen Formel (I)
A-X-Y-Z-R₁ (I)
wobei A eine Carboxygruppe umfasst oder nicht vorhanden ist;
X ein Polyol umfasst, wobei ein oder mehrere Polyolhydroxyle durch Acyl substituiert sind;
Y -C(=O)-, -C(=S)- umfasst oder nicht vorhanden ist;
Z O, S oder NH umfasst; und
R₁ einen Polyether umfasst;
zur Verwendung zum Hemmen oder Reduzieren von Atherosklerose oder einer atherosklerotischen Entwicklung in einem Tier.

## Revendications

1. Utilisation d'un composé selon la formule chimique (I)
A-X-Y-Z-R₁ (I)
dans laquelle A comprend un groupe carboxyle ou est absent ;
X comprend un polyol, dans laquelle un ou plusieurs hydroxyles polyol sont substitués par un acyle ;
Y comprend -C(=O)-, -C(=S)- ou est absent ;
Z comprend O, S ou NH ; et
R₁ comprend un polyéther ;
pour la préparation d'un médicament destiné à l'inhibition ou à la réduction de l'athérosclérose ou d'un développement athérosclérotique chez un animal.

2. Utilisation selon la revendication 1, dans laquelle l'acyle polyol comprend un ou plusieurs acides gras.

3. Utilisation selon la revendication 1 ou 2, dans laquelle le polyol comprend un alkyle polyol (C₂-C₂₀).

4. Utilisation selon l'une quelconque des revendications 1 à 3, dans laquelle le polyol comprend entre environ 2 et 20 groupes hydroxyle.

5. Utilisation selon l'une quelconque des revendications 1 à 4, dans laquelle le polyol est substitué avec un ou plusieurs groupes acyle.

6. Utilisation selon l'une quelconque des revendications 1 à 5, dans laquelle le polyol comprend un alkyle polyol monocarboxylique ou dicarboxylique (C₂-C₂₀) substitué avec entre environ 1 et 10 groupes hydroxyle.

7. Utilisation selon l'une quelconque des revendications 1 à 6, dans laquelle le polyol comprend un ou plusieurs acides parmi l'acide mucique, l'acide malique, l'acide citromalique, l'acide alkylmalique, les dérivés de l'acide hydroxyglutarique, les acides alkylglutariques, l'acide tartrique et l'acide citrique.

8. Utilisation selon l'une quelconque des revendications 1 à 7, dans laquelle le polyol comprend un ou plusieurs composés parmi l'acide 2,2-(bis(hydroxyméthyle) propanoïque, la tricine et un saccharide.

9. Utilisation selon l'une quelconque des revendications 1 à 8, dans laquelle le polyéther comprend entre environ 2 et 150 unités d'alkylène oxyde.

10. Utilisation selon l'une quelconque des revendications 1 à 9, dans laquelle chaque unité d'alkylène oxyde comprend un alkylène oxyde (C₂-C₄) droit ou ramifié.

11. Utilisation selon l'une quelconque des revendications 1 à 10, dans laquelle le polyéther comprend un groupe terminal alcoxyle.

12. Utilisation selon l'une quelconque des revendications 1 à 11, dans laquelle le polyéther est lié au polyol via un groupe de liaison comprenant un ester, un thioester ou un amide.

13. Utilisation selon l'une quelconque des revendications 1 à 12, dans laquelle le polyéther répond à la formule chimique
R₅-(R₆-O-)ₐ-R₆-Q- (II),
dans laquelle
R₅ comprend un groupe droit ou ramifié (C₁-C₂₀) alkyle, -OH, -OR₇, -NH₂, -NHR₇, -NHR₇R₈, -CO₂H, -SO₃H (sulfo), -CH₂-OH, -CH₂-OR₇, -CH₂-O-CH₂-R₇, -CH₂-NH₂, -CH₂-NHR₇, -CH₂-NR₇R₈, -CH₂CO₂H, -CH₂SO₃H ou -O-C(=O)-CH₂-CH₂-C(=O)-O- ;
R₆ comprend un alkylène bivalent (C₂-C₁₀) droit ou ramifié ;
chaque groupe R₇ et R₈ comprend, indépendamment, un alkylène (C₁-C₆) droit ou ramifié ;
Q comprend -O-, -S- ou -NR₇ ; et
a comprend un entier compris entre environ 2 et 110, ces nombres compris.

14. Utilisation selon l'une quelconque des revendications 1 à 13, dans laquelle le polyéther comprend un polyéthylène glycol comprenant un groupe terminal méthoxyle.

15. Utilisation selon l'une quelconque des revendications 2 à 14, dans laquelle le ou les acides gras comprennent un ou des acides gras (C₂-C₂₄).

16. Utilisation selon l'une quelconque des revendications 2 à 15, dans laquelle le ou les acides gras comprennent un ou plusieurs acides parmi les acides caprylique, caprique, laurique, myristique, myristoléique, palmitique, palmitoléique, stéarique, oléique, linoléique, arachidique, béhénique et érucique.

17. Utilisation selon l'une quelconque des revendications 1 à 16, dans laquelle le composé selon la formule (I) présente une structure chimique 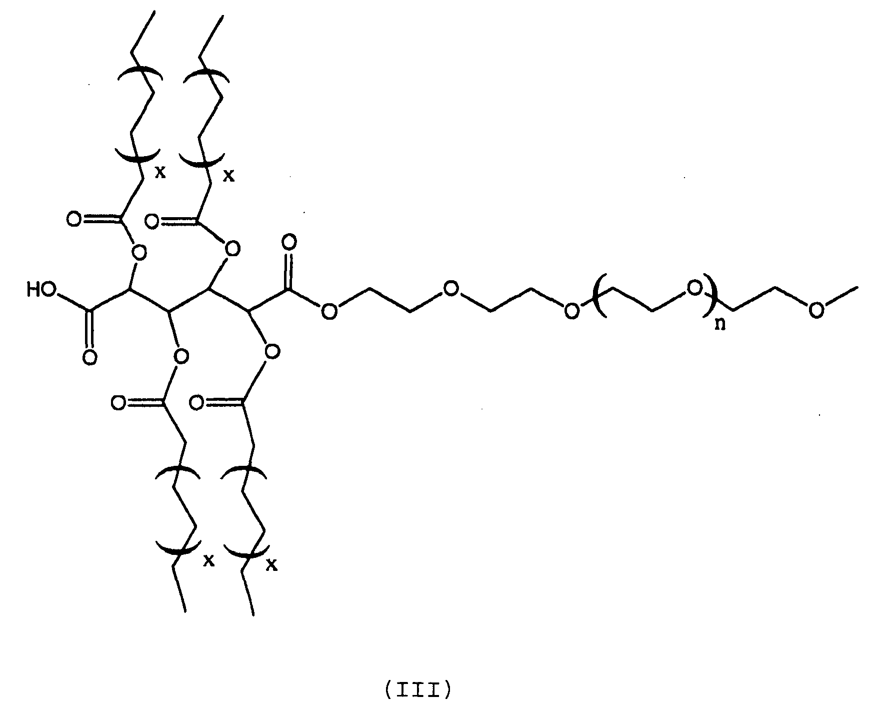 dans laquelle chaque x est, indépendamment, 1, 2, 3 ou 4 ; et n est environ égal à 36.

18. Utilisation selon l'une quelconque des revendications 1 à 17, dans laquelle le composé selon la formule (I) est fourni sous forme d'une formulation de nanoparticules.

19. Utilisation selon la revendication 1, dans laquelle l'animal est un être humain.

20. Composé selon la formule chimique (I)
A-X-Y-Z-R₁ (I)
dans lequel A comprend un groupe carboxyle ou est absent ;
X comprend un polyol, dans lequel un ou plusieurs hydroxyles polyol sont substitués par un acyle ;
Y comprend -C(=O)- ou -C(=S)-, ou est absent ;
Z comprend O, S ou NH ; et
R₁ comprend un polyéther ;
pour une utilisation destinée à l'inhibition ou à la réduction de l'athérosclérose ou d'un développement athérosclérotique chez un animal.
